# EUROPÄISCHE PATENTSCHRIFT

(11) **EP 1 601 385 B1**
(45) Veröffentlichungstag und Bekanntmachung des Hinweises auf die Patenterteilung: **31.10.2007**
(21) Anmeldenummer: 04719344.6
(22) Anmeldetag: 11.03.2004
(51) Int. Cl.: A61L 2/10, A61L 2/26, A61L 2/28

(54) **VORRICHTUNG ZUM ENTKEIMEN VON INSTRUMENTEN**
DEVICE FOR STERILISING INSTRUMENTS
DISPOSITIF DESTINE AU DEGERMAGE D'INSTRUMENTS

(30) Priorität: 12.03.2003 CH 4042003
(43) Veröffentlichungstag der Anmeldung: 07.12.2005
(73) Patentinhaber: AMESEDER, Anton, CH-9400 Rorschach (CH)
(72) Erfinder: AMESEDER, Anton, CH-9400 Rorschach (CH)
(74) Vertreter: Kulhavy, Sava
(86) Internationale Anmeldenummer: PCT/CH2004/000145
(87) Internationale Veröffentlichungsnummer: WO 2004/080495

(56) Entgegenhaltungen:
- EP-A- 1 038 536
- CH-A- 670 568
- DE-A- 10 048 071
- US-B1- 6 429 438

## Beschreibung

Die vorliegende Erfindung betrifft eine Vorrichtung zum Entkeimen von Instrumenten, insbesondere von Zahnbürsten, durch ultraviolette Strahlung, welche durch eine Strahlungsquelle erzeugt werden kann, wobei diese Vorrichtung so ausgeführt ist, dass der Betrieb der Strahlungsquelle überwacht werden kann, mit einem Innenteil, welcher eine längliche Hinterwand aufweist, über welche der Innenteil an einer Wand angebracht sein kann, und mit einem Gehäuse, welches dem Innenteil zugeordnet sein kann.

In CH-PS 670 568 ist eine Vorrichtung dieser Gattung offenbart. Das Gehäuse ist bei dieser vorbekannten Vorrichtung vom Innenteil wegnehmbar. Im Innenteil, befindet sich der elektrische Teil dieser Vorrichtung, welcher aus einer Quelle mit elektrischer Energie gespeist werden muss. Diese Quelle ist an den elektrischen Teil im Innenteil fest angeschlossen. Die Vorrichtung der vorliegenden Art wird oft im Badezimmer oder dgl. aufgestellt. Da das Gehäuse vom Innenteil jederzeit wegnehmbar ist, besteht die Möglichkeit, dass der Benützer dieser Vorrichtung Bestandteile des elektrischen Teiles der vorbekannten Vorrichtung berührt kann. Dies kann einen elektrischen Schlag zur Folge haben. Ferner gilt bei der vorbekannten Vorrichtung als nachteilig, dass die Intensität der UV-Strahlung wegen Alterung der Strahlungsquelle abnehmen kann. Nach einer bestimmten Betriebsdauer kann die Intensität der UV-Strahlung dermassen abnehmen, dass eine ausreichende Entkeimung der Instrumente nicht mehr gewährleistet ist. Ferner kann es auch vorkommen, dass die UV-Lampe ausfällt. Auch in diesem Fall ist die Entkeimung der Instrumente nicht mehr gewährleistet.

Die Aufgabe der vorliegenden Erfindung ist, diese sowie noch weitere Nachteile des Standes der Technik zu beseitigen.

Diese Aufgabe wird bei der Vorrichtung der eingangs genannten Gattung erfindungsgemäss so gelöst, wie dies im kennzeichnenden Teil des Patentanspruchs 1 definiert ist.

Nachstehend werden Ausführungsformen der vorliegenden Erfindung anhand der beiliegenden Zeichnungen näher erläutert. Es zeigt:
Fig. 1 perspektivisch die vorliegende Vorrichtung mit einem Frontdeckel,
Fig. 2 perspektivisch die Vorrichtung aus Fig. 1, bei welcher der Frontdeckel auf seiner Frontwand liegt und bei welcher ein Innenteil der Vorrichtung aufrecht stehend dargestellt ist,
Fig. 3 in einem vertikalen Längsschnitt eine Platte des Innenteiles aus Fig. 2, welche den elektronischen Teil der vorliegenden Vorrichtung trägt,
Fig. 4 ein Blockschaltbild einer ersten Ausführung des elektronischen Teiles der vorliegenden Vorrichtung,
Fig. 5 ein Blockschaltbild eines Abschnittes des elekronischen Teiles, welcher zu einer ersten Art der Signalisieren der Arbeitsweise der vorliegenden Vorrichtung ausgeführt ist,
Fig. 6 ein Blockschaltbild eines Abschnittes des elektronischen Teiles, welcher zu einer zweiten Art der Signalisierung der Arbeitsweise der vorliegenden Vorrichtung ausgeführt ist,
Fig. 7 ein Blockschaltbild einer zweiten Ausführung des elektronischen Teiles der vorliegenden Vorrichtung,
Fig. 8 in einem vertikalen Schnitt einen Halteeinrichtung für die Instrumente,
Fig. 9 perspektivisch einen Grundkörper der Halteeinrichtung aus Fig. 8 und
Fig. 10 perspektivisch einen Kamm der Halteeinrichtung.

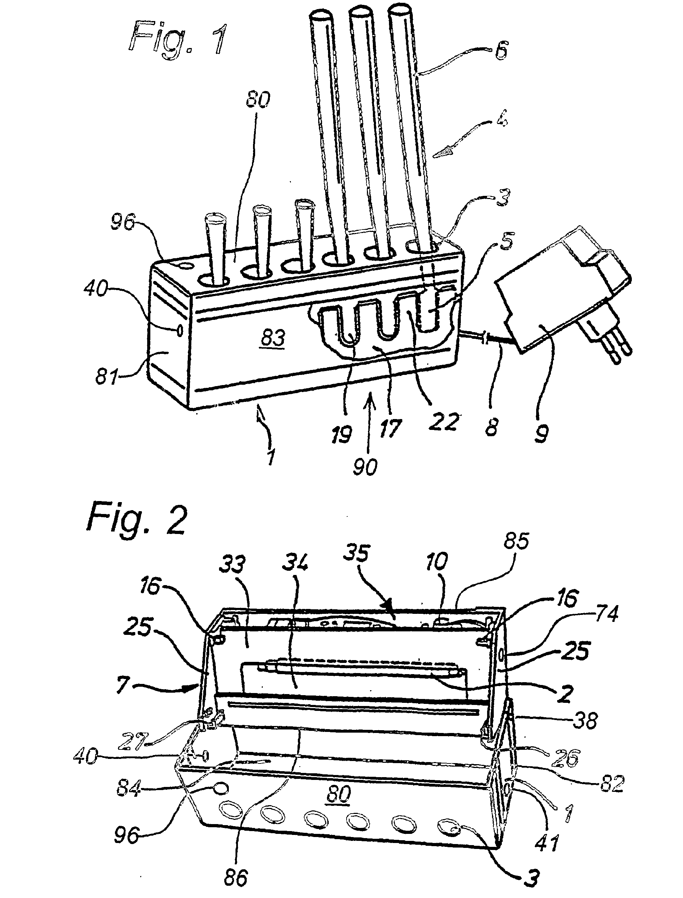

Die vorliegende Entkeimungsvorrichtung umfasst unter anderem ein optisch ansprechendes längliches Gehäuse 1 (Fig. 1 und 2), welches vorzugsweise aus einem Kunststoff ist. Dieses Gehäuse wird im Nachstehenden auch als Frontdeckel 1 der vorliegenden Vorrichtung bezeichnet. Dieses Gehäuse 1 weist, wie dies aus Fig. 1 und 2 ersichtlich ist, eine Oberwand 80, Seitenwände 81 und 82, eine Frontwand 83 und eine Bodenwand 84 auf. In der Oberwand 80 ist eine Reihe von Öffnungen 3 ausgeführt, welche sich in der Längsrichtung des Gehäuses 1 erstreckt. Durch die Öffnungen 3 können die zu entkeimenden Instrumente, im vorliegenden Fall sind dies Zahnbürsten 4, hindurchgesteckt werden. In Fig. 1 ist auch ein Stecknetzgerät 9 dargestellt, welches über ein Kabel 8 an den elektrischen Teil der vorliegenden Vorrichtung angeschlossen ist.

Die vorliegende Entkeimungsvorrichtung umfasst ferner einen Innenteil 7 (Fig. 2), an welchem das Gehäuse 1 schwenkbar angeschlossen ist. Der Innenteil 7 weist einen im wesentlichen planen Grundkörper 85 auf. Dieser Grundkörpers 85 stelle eine Wand dar, welche die Hinterwand der Vorrichtung darstellt. Über diese Hinterwand 85 kann die Vorrichtung beispielsweise an einer Zimmerwand angebracht sein. An die jeweilige Endkante der länglichen Hinterwand 85 schliesst sich je eine Wange 25 an, welche senkrecht zur Ebene der Hinterwand 85 stehen und sich von dieser Hinterwand 85 nach vorne erstrecken. Zwischen den Wangen 25 erstreckt sich eine Trennwand 33, welche sich in einem Abstand von der Hinterwand 85 befindet und welche zur Hinterwand 85 parallel verläuft.

An der Aussenseite der jeweiligen Wange 25 ist je ein Scharnier 38 vorhanden (Fig. 2). Die Scharniere 38 ermöglichen den bereits erwähnten schwenkbaren Anschluss des Frontdeckels 1 an den Innenteil 7. Das jeweilige Scharnier 38 weist im dargestellten Beispiel einen Zapfen auf, welcher an der Aussenseite der betreffenden Wange 25 des Innenteiles 7 angeformt ist, sowie eine diesen Zapfen aufnehmen Öffnung in einer der Seitenwände 81 bzw. 82 des am Innenteil 7 wegschwenkbar angebrachten Frontdeckels 1. Je eines dieser Scharniere 38 wirkt somit zwischen der Aussenseite einer der Innenteilwangen 25 und einer der Seitenwände 81 bzw. 82 des Gehäuses 1. In Fig. 1 ist das Gehäuse 1 auf dem Innenteil 7 eingeschwenkt und eingerastet dargestellt.

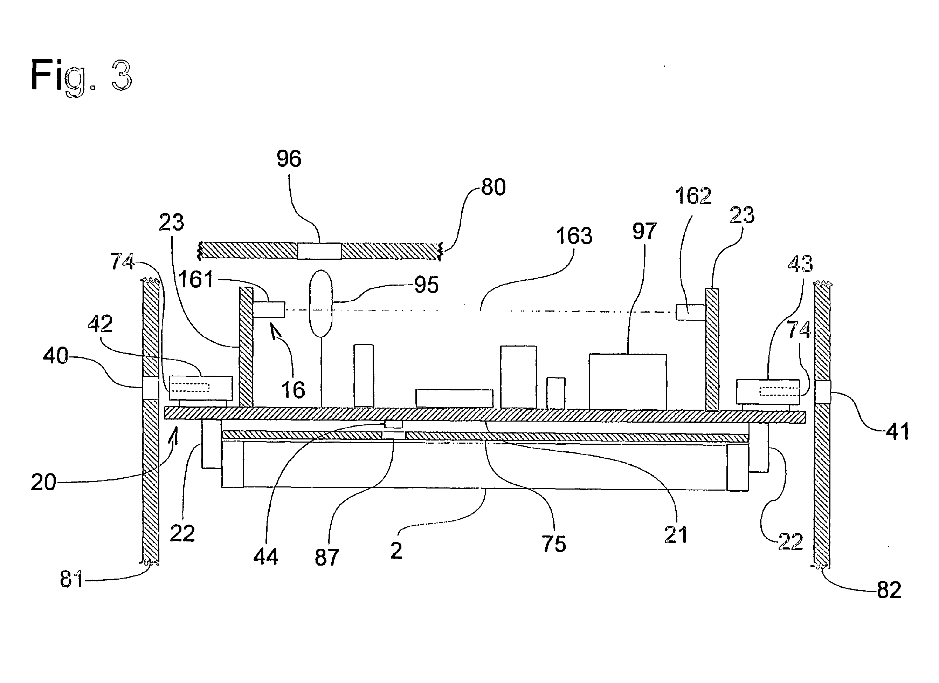

Im Hohlraum bzw. im Aufnahmeschacht 35, welcher zwischen der Hinterwand 85 des Innenteiles 7 und der Trennwand 33 vorhanden ist, und etwa in der Mitte der Höhe der Wangen 25 ist ein Träger 20 (Fig. 3) für den elektrischen Teil 10 der vorliegenden Vorrichtung im Innenteil 7 einrastbar angeordnet. Dieser Träger 20 weist einen im wesentlichen planen bzw. streifenförmigen Grundkörper 21 auf. Dieser Grundkörper 21 ist als eine Platine aus einem elektrisch isolierenden Material ausgeführt und er trägt die gesamte Elektronik einschliesslich der Steuerungseinrichtung 10 und der Lichtquelle 2.

In der Trennwand 33 ist eine längliche Öffnung 34 ausgeführt, welche sich parallel zur Lichtquelle 2 erstreckt. Durch diese Lichtöffnung 34 kann das von der Lichtquelle 2 abgestrahlte in jenen Bereich der vorliegenden Vorrichtung gelangen, wo sich die Instrumente 4 befinden. Im Bereich der jeweiligen Endpartie des Trägers 20 ist je ein Halter 23 (Fig. 2) angeordnet. Der jeweilige Halter 23 ist im dargestellten Fall als ein flaches Stück aus einem ebenfalls isolierenden Material ausgeführt. Dieses Materialstück 23 steht praktisch senkrecht zum Grundkörper 21 des Trägers 20 und er befindet sich oberhalb des Trägers 20 des Innenteiles 7.

Die vorliegende Entkeimungsvorrichtung umfasst ferner eine Halteeinrichtung 90 (Fig. 1, 8 und 9) für die Instrumente 4. Diese Halteeinrichtung 90 ist der Vorder- bzw. Aussenseite der Trennwand 33 des Innenteiles 7 in einer an sich bekannten Weise zugeordnet. Dies kann beispielsweise mit Hilfe von Führungen 27 (Fig. 2) erfolgen, von welchen je eine an der Innenseite der jeweiligen Wangen 25 angebracht ist. Die Halteeinrichtung 90 weist einen im Querschnitt im wesentlichen L - förmigen Grundkörper 91 mit den Schenkeln 92 und 93 auf. Der erste dieser L-Schenkel 92 verläuft praktisch horizontal und der zweite L-Schenkel 93 erstreckt sich vom ersten Schenkel 92 empor. Die einander gegenüberliegenden Randpartien des horizontalen Schenkels 92 können in die Führungen 27 eingeschoben werden.

Im Uebergangsbereich zwischen den L-Schenkeln 92 und 93 ist eine längliche Wanne 94 vorhanden, welche sich auf dem unteren bzw. horizontalen L-Schenkel 92 befindet und welche sich praktisch zwischen den Wangen 25 erstreckt. Im jeweiligen Endbereich der Wanne 94 ist je eine schlitzartige Führung 29 vorhanden, welche sich vom Boden der Wanne 94 aufwärts, d.h. vertikal und praktisch parallel zum zweiten L-Schenkel 93 erstrecken. Diese Halteeinrichtung 90 umfasst ferner einen Kamm 17, dessen einander gegenüberliegenden Randpartien 32 in die jeweilige Führung 98 bzw. 99 eingerührt werden können.

Folglich befindet sich dieser Kamm 17 in einem Abstand vom vertikalen L-Schenkel 93 und er verläuft praktisch parallel zu diesem Schenkel 93. Nach dem Einführen des Aufnahmekamms 17 in die Halteeinrichtung 90 wird diese Einrichtung 90 selbst über ihren horizontalen L-Schenkel 92 nach Art eines Einschubs der Trennplatte 33 zugeordnet, indem die Ränder des horizontalen L-Schenkels 92 in die Führungen 27 an den Wangen 25 eingeschoben werden.

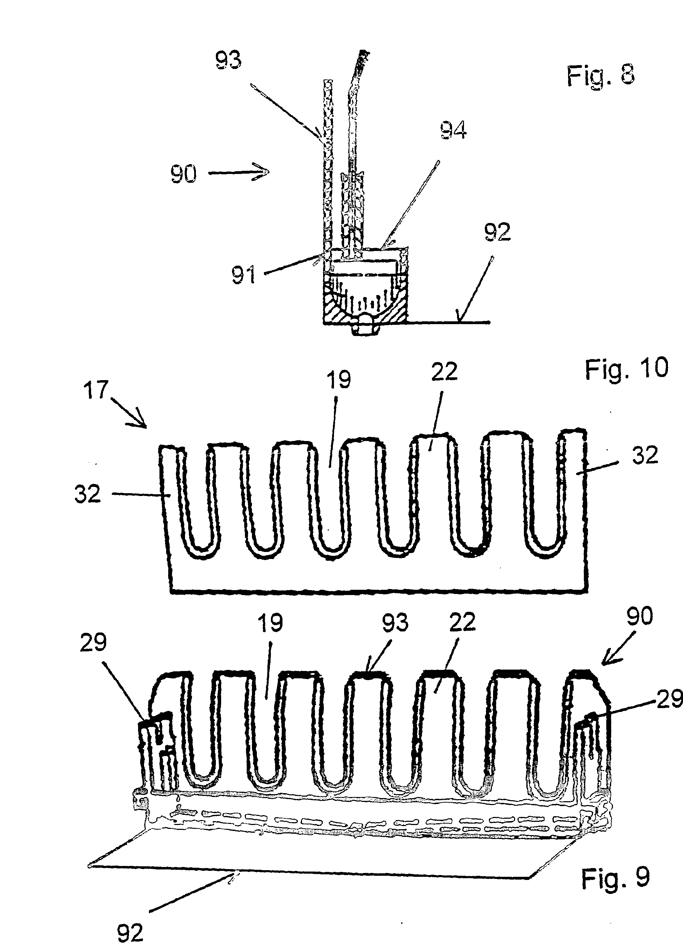

Der Kamm 17 ist zur Aufnahme der zu entkeimenden Instrumente 4 bestimmt und ausgebildet. Im Kamm 17 sind von oben her vertikal verlaufende Einschnitte 19 ausgeführt (Fig. 1 und 10), deren Breite der Breite des Kopfes 5 einer Zahnbürste 4 entspricht. Zwischen den Einschnitten 19 befinden sich Zinken 22 des Kammes 17. Der jeweilige Einschnitt 19 öffnet sich gegen eine der Öffnungen 3 in der Oberwand 80 des Behälters 1 hin. Der Bürstenkopf 5 ist so orientiert, dass die Borsten desselben gegen die Aussenseite der Trennwand 33 gerichtet sind. In diesem Bereich ist die Trennwand des Innenteiles 7 mit einem Fenster 34 versehen. Der Griff 6 der Zahnbürsten 4 ragt durch die Öffnungen 3 aus dem Gehäuse 1 heraus. Der Abstand des Kammes 17 vom Grundkörper 91 entspricht etwa der Dicke des Griffes der Zahnbürste 4.

Sollte die Dicke des Griffes des Instrumentes 4 erheblich sein, dann ist es zweckdienlich, den vertikalen L-Schenkel 93 der Halteeinrichtung 90 ebenfalls mit den im Zusammenhang mit dem Kamm bzw. Rechen 17 bereits beschriebenen Einschnitten 19 zu versehen (Fig. 10). In einem solchen Fall ist es zweckdienlich, wenn die Einschnitte 19 bzw. die Zinken 22 der Platte 93 mit den Einschnitten 19 bzw. Zinken 22 des Rechens 17 ausgerichtet sind bzw. mit diesen fluchten.

Die einander gegenüberliegenden Endabschnitte 24 des länglichen Trägers 20, welche an der Aussenseite der Halter 23 liegen, tragen je eine Buchse 42 bzw. 43, deren Längsachsen parallel zur Ebene des Grundkörpers 21 verlaufen. Diese Buchsen 42 und 43 sind für die Aufnahme eines handelsüblichen Steckers am Steckernetzteil 9 ausgeführt und sie öffnen sich in einander entgegengesetzten Richtungen, und zwar nach aussen hin. Wenn das Gehäuse 1 auf dem Innenteil 7 aufgeschwenkt ist, dann stehen die sich nach aussen hin öffnenden Mündungen 74 der Buchsen 42 und 43 bestimmten Stellen an den Seitenwänden 81 und 82 des Gehäuses 1 gegenüber. Diese Stellen an den Gehäusewänden 81 und 82 sind mit je einer entsprechenden Öffnung 40 bzw. 41 versehen. Diese Öffnungen 40 und 41 sind so ausgeführt, dass der Stecker des Steckernetzgerätes 9 durch die betreffende Öffnung 40 bzw. 41 hindurchgesteckt und in die betreffende Buchse 42 bzw. 43 eingesteckt werden kann.

Die Verwendung der zwei genannten Buchsen bietet den Vorteil, dass der Stecker des Netzgerätes 9 von jener Seite her in die vorliegende Vorrichtung eingeführt werden kann, welche unter den gegebenen Bedingungen als vorteilhafter erscheint. Ausserdem wirkt der eingesteckte Stecker auch als Sicherung. Einerseits wirkt er als Sicherung gegen ein unabsichtliches Öffnen der Vorrichtung und zweitens wirkt er als Sicherung gegen ein Öffnen der Vorrichtung, solange diese noch ans Netz angeschlossen ist.

Der elektrische Teil der vorliegenden Vorrichtung umfasst unter anderem auch einen Sensor 16, welcher das Einführen eines Instrumentes 4 in das Gehäuse 1 detektieren soll. Dieser Sensor 16 ist im vorliegenden Fall als eine Lichtschranke ausgeführt. Diese Lichtschranke umfasst einen Lichtsender 161 und einen Lichtempfänger 162, zwischen welchen sich eine Lichtstrecke 163 befindet. Die Lichtschranke 16 kann auch mit Infrarotlicht arbeiten, sodass eine solche Lichtschranke einen Infrarotsender und einen Infrarotempfänger umfasst. Die Lichtquelle 161 der Lichtschranke 16 ist an einem der Halter 23 und der Lichtempfänger 162 der Lichtschranke 16 ist am anderen Halter 23 befestigt, und zwar in einem Abstand oberhalb der Platine 20. Dieser Abstand ist grösser als die Länge des Kopfes 5 der Zahnbürste 4. Die Lichtquelle 161 und der Lichtempfänger 162 sind an je einer der Grossflächen der Halter 23 angebracht, welche einander zugewandt sind.

Wie bereits erwähnt, ist das Gehäuse bzw. der Deckel 1 am U-förmigen Innenteil 7 schwenkbar angebracht. Während dem Betrieb der vorliegenden Einrichtung umgibt bzw. verdeckt der Frontdeckel 1 den Innenteil 7. Die Lichtschranke 16 ist oberhalb jenem Raum angeordnet, in welchem sich die Köpfe der in die Vorrichtung eingeführten Zahnbürsten 4 während der Entkeimung befinden. Die Lichtschranke 16 ist zugleich vor dem Kamm 17 so angeordnet, dass die Borsten der Köpfe 5 der Zahnbürsten 4 den Lichtstrahl 163 zwischen der Lichtquelle 161 und dem Lichtempfänger 162 der Lichtschranke 16 vorübergehend unterbrechen, wenn eine Zahnbürsten 4 durch eine der Öffnungen 3 des Dekkels 1 in das Innere dieser Vorrichtung eingeführt wird.

Die Lichtquelle 2 der vorliegenden Vorrichtung ist als eine ultraviolette Strahlen erzeugende Quelle ausgeführt. Im vorliegenden Fall handelt es sich um eine UV-C Röhre. Auf der Unterseite des Grundkörpers 21 des Träger 20 sind zwei Fassungen 22 für die Endpartien der UV-Röhre 2 befestigt. Diese Fassungen 22 sind so angeordnet, dass sich die UV-Lichtquelle 2 auf der Höhe der Borsten des Kopfes 5 der Zahnbürsten 4 befindet, welche in der Vorrichtung eingesteckt sind. Die Zahnbürsten 4 sind im Gehäuse 1 so gehalten, dass ihre Borsten zur UV-Lichtquelle 2 hin zeigen. Beim Einführen einer Zahnbürste in das Gehäuse 1 wird die Lichtschranke 16 betätigt und die UV-Lichtquelle 2 wird zum Betrieb während einer vorgegebenen Zeitspanne veranlasst. Die Zahnbürsten 4 oder andere Instrumente sind damit den ultravioletten Strahlen ausgesetzt und werden dadurch keimfrei gemacht bzw. keimfrei gehalten..

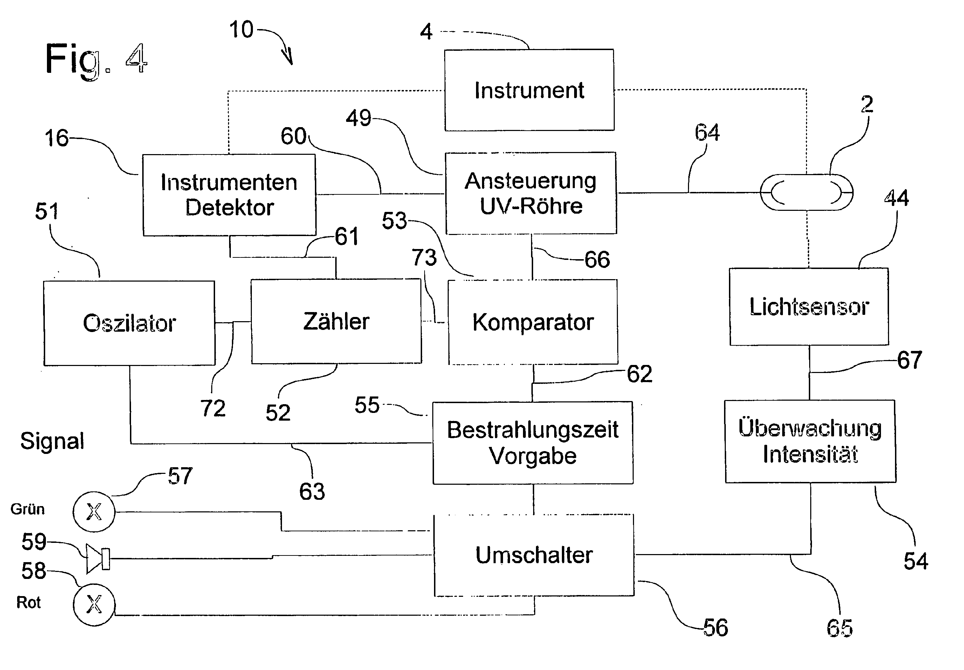

Der elektrische Teil der vorliegenden Vorrichtung umfasst auch eine elektronische Steuerungseinrichtung 10, welche am Träger 20 ebenfalls angebracht ist und sich somit zusammen mit der Lichtquelle 2 auf der gemeinsamen Platine 21 befindet. In Fig. 4 ist die Topologie einer ersten Ausführung dieser Steuerungseinrichtung 10 der vorliegenden Vorrichtung schematisch dargestellt. Fig. 4 zeigt auch den bereits genannten Lichtsensor 16 für die Detektion der Instrumente 4. Die Instrumente 4 sind andererseits den Strahlen der UV-Röhre 2 ausgesetzt. Die Wege des Lichtes sind in Fig. 4 mit punktierten Linien angedeutet.

Zwischen dem Träger 20 und der UV-Röhre 2 befindet sich ein streifenförmiger Reflektor 75. Dieser Reflektor 75 erstreckt sich zwischen den Fassungen 22 für UV-Röhre 2. Der Reflektor 75 ist aus einem elektrisch isolierendem Material und er hat zumindest eine verspiegelte, elektrisch leitende Oberfläche. Diese verspiegelte Oberfläche ist der UV-Röhre 2 zugewandt. In dem Reflektor 75 ist eine durchgehende Öffnung 87 ausgeführt. Diese Öffnung 87 befindet sich in jenem Bereich des Reflektors 75, wo der Tageslichtsensor 44 am Träger 20 angeordnet ist. Durch diese Öffnung strahlt die von der Röhre 2 ausgesendete Strahlung ungehindert auf den sich dahinter befindlichen Tageslichtsensor 44.

An einen ersten Ausgang des Instrumentendetektors 16 ist eine Ansteuerungselektronik 49 für die UV-Röhre 2 über eine Leitung 60 angeschlossen. Auch diese Ansteuerungselektronik 49 wird aus dem Steckernetzgerät 9 gespiesen und sie dient zur Wandlung der Speisespannung vom Steckernetzteil 9 in die für den Betrieb der Röhre 2 notwendige elektrische Spannung mit der erforderlichen Frequenz. Diese Ansteuerungselektronik 49 ist über eine Speiseleitung 64 an die UV-Röhre 2 angeschlossen.

Die Schaltungsanordnung 10 umfasst ferner einen Lichtsensor 44. Dieser Lichtsensor 44 ist zur Erfassung von Licht vorgesehen, welches aus der UV-Röhre 2 austritt. Im vorliegenden Fall ist nicht nur die Tatsache von Bedeutung, ob die Röhre 2 UV-Licht überhaupt ausstrahlt, sondern auch, wie gross die Intensität dieses UV-Lichtes ist. Der Lichtsensor 44 ist somit auch so ausgeführt, dass der Ausgang desselben ein Signal entsprechend der Intensität des empfangenen UV-Lichtes abliefern kann. Im vorliegenden Fall ist der Sensor 44 derart, dass er normalerweise zur Messung von Tageslicht bestimmt ist. Im dargestellten Fall ist der Lichtsensor 44 in einer Öffnung in der Platine 21 eingelassen und zwar derart, dass seine Empfangsöffnung gegen die sich an der anderen Seite bzw. sich unterhalb der Platine 20 befindliche UV-Röhre 2 hin gerichtet ist (Fig. 3).

Eine Messleitung 67 verbindet den Ausgang des Lichtsensors 44 mit dem Eingang einer Einrichtung 54 zur Überwachung der Intensität der UV-Strahlen. Die Intensität des von der Röhre 2 abgestrahlten UV-Lichtes ist nämlich gleich nach der Einschaltung der UV-Röhre 2 noch nicht so hoch, wie diese für die Entkeimung der Instrumente erforderlich ist. Die Intensität der UV-Strahlung erreicht erst nach einer bestimmten Verzögerung den für die Entkeimung erforderlichen Wert. Erst nachdem die Intensität der UV-Strahlung diesen Wert erreicht hat, beginnt die für die Entkeimung erforderliche Zeitspanne zu laufen. An einen der Ausgänge der Ueberwachungsvorrichtung 54 ist ein Umschalter 56 über eine Leitung 65 angeschlossen. Der Umschalter 56 ist unter anderem auch an eine Einrichtung zur Signalisierung des Betriebes der UV-Röhre 2 bzw. des Verlaufes des Entkeimungsprozesses angeschlossen. Diese Einrichtung ist im nachstehenden näher beschrieben.

Zur Bestimmung der Länge der genannten Bestrahlungszeitspanne dient eine weitere Gruppe der Steuerelektronik 10. Diese Gruppe umfasst eine Einrichtung 55 zur Vorgabe der Länge der Bestrahlungszeit, einen Oszilator bzw. eine Quelle von Impulsen 51 und einen an den Ausgang dieser Quelle 51 über eine Leitung 72 angeschlossenen Zähler 52, welcher über eine Signalleitung "Bestrahlungszeit" 73 an einen der Eingänge eines Komparators 53 angeschlossen ist. Der Betrieb des Zählers 52 wird über eine Leitung 61 gesteuert, welche einen zweiten Ausgang des Instrumentendeteldors 16 mit einem zweiten Eingang des Zählers 52 verbindet. Ein erster Ausgang des bereits erwähnten Umschalters 56 ist an einen ersten Eingang der Schaltung "Bestrahlugsvorgabe" 55 angeschlossen. Ein erster Ausgang der Vorgabeeinrichtung 55 ist an einen zweiten Eingang des Komparators 53 angeschlossen. Ein zweiter Ausgang der Bestrahlungsvorgabe 55 ist an einen Steuereingang des Oszillators 51 angeschlossen. Der Ausgang des Komparators 53 steuert über eine Leitung 66 den Betrieb der Ansteuerungseinrichtung 49 für die UV-Röhre 2. Im in Fig. 4 dargestellten Fall ist die Vorrichtung so ausgeführt, dass die Bestrahlungszeit in der Bestrahlungsvorgabe 55 fest eingestellt ist.

Beim Einschieben in die Vorrichtung unterbricht der Bürstenkopf 5 des ersten Instrumentes 4 den Lichtstrahl in der Lichtstrecke 163 des Instrumentendetektors 16. Dieser Instrumentendetektor 16 sendet einen Steuerimpuls über die Steuerleitung 60 zur Einrichtung "Ansteuerung UV-Röhre" 49. Gleichzeitig wird der Zähler 52 durch den Instrumentendetektor 16 über die Steuerleitung 61 auf Null gesetzt. Die Einrichtung "Ansteuerung UV-Röhre" 49 beginnt, die UV-Röhre 2 mit für den Betrieb dieser Röhre 2 erforderlichen Energie zu versorgen. Damit wird die UV-Röhre 2 gezündet und beginnt UV-Licht abzustrahlen. Der Lichtsensor 44 misst die Intensität der von der Röhre 2 abgegebenen Strahlung und legt sie auf die Messleitung 67. Die Einrichtung "Überwachung Intensität" 54 prüft über einen Schwellwertschalter, ob die Intensität für die Entkeimung der Instrumente 4 ausreichend ist.

Normalerweise steigt die Intensität des durch die Röhre 2 abgestrahlten UV-Lichtes von einem Anfangswert, welcher unterhalb jenes Wertes liegt, der für die Entkeimung der Instrumente 4 erforderlich ist, innerhalb von wenigen Sekunden auf diesen vorgeschriebenen Wert. Während dieser Anfangsphase liegt der Wert des Signals am Ausgang des Lichtsensors 44 unterhalb einem Pegel, oberhalb welchem die Intensität des UV-Lichtes für die Entkeimung der Instrumente 4 ausreichend ist. Nach Ablauf dieser Anfangsphase, d.h. nachdem die Intensität des durch die Röhre 2 abgegebenen UV-Lichtes den vorgeschriebenen Pegelwert erreicht bzw. diesen überschritten hat, meldet dies die Einrichtung "Überwachung Intensität" 54 über die Signalleitung 65 dem Umschalter 56. Dieser Umschalter 56 gibt ein Signal an die Bestrahlungsvorgabe 55 ab, womit die fest eingestellte Bestrahlungszeit zu laufen beginnt.

Die Bestrahlungsvorgabe 55 liefert eine Information über die fest eingestellte Länge der erforderlichen Bestrahlungszeit an den Komparator 53 ab, und zwar in Form einer der gewünschten Länge der Bestrahlungszeit entsprechenden Anzahl von Impulsen. Der Oszillator 51 liefert Impulse an den Zähler 52, welcher diese kumuliert und welcher eine Auskunft über die Anzahl der vom Oszillator 51 empfangenen Impulse an den Komparator 53 liefert. Wenn die Anzahl der Impulse vom Zähler 52 der Anzahl der Impulse aus der Vorgabe 55 gleicht, liefert der Komparator 53 ein Signal an die Ansteuerung 49 ab, welche die UV-Röhre 2 ausser Betrieb setzt.

Wird ein Instrument 4 in den Auffangbehälter 1 eingeführt, während sich andere und zuvor eingelegte Instrumente 4 schon im Entkeimungsprozess befinden, wird das Einführen des weiteren Instrumentes 4 in gleicher Weise wie die vorherigen Instrumente 4 vom Instrumentendetektor 16 durch einen Unterbruch in der Lichtstrecke 163 zwischen den Teilen 161 und 162 des Instrumentendetektors 16 erkannt. Mit einem Signal über die Steuerleitung 61 erfolgt eine Rückstellung des Zählers 52, sodass die vorgeschriebene Entkeimungszeit nach dem Einschieben eines weiteren Instrumentes 4 von neuem zu laufen beginnt. Dadurch erreicht die Entkeimungszeit für das zuletzt eingeschobene Instrument 4 den vollen von der "Bestrahlungszeit Vorgabe" 55 vorgegebenen Wert. Jene Instrumente 4, welche sich in der Vorrichtung bereits befinden, werden weiter und somit um so länger entkeimt.

Beispielsweise wegen einem länger dauernden Betrieb kann die Intensität des durch die Röhre 2 abgestrahlten Lichtes unter den für die Entkeimung erforderlichen Wert fallen. Die Röhre 2 brennt in einem solchen Fall weiter, weil sie über die Leitung 64 mit Energie versorgt wird. Der Benützer der Vorrichtung könnte es somit gar nicht merken, dass die Instrumente 4 nicht mehr ausreichend entkeimt werden. Um dies zu verhindern, ist die Einrichtung "Bestrahlungzeitvorgabe" 55 so ausgeführt, dass die Bestrahtungszeitvorgabe veränderbar ist. Zweckmässig ist, dass die Bestrahlungszeit entsprechend der Abnahme der Intensität des UV-Lichtes verlängert werden kann, und dass diese Verlängerung automatisch erfolgt.

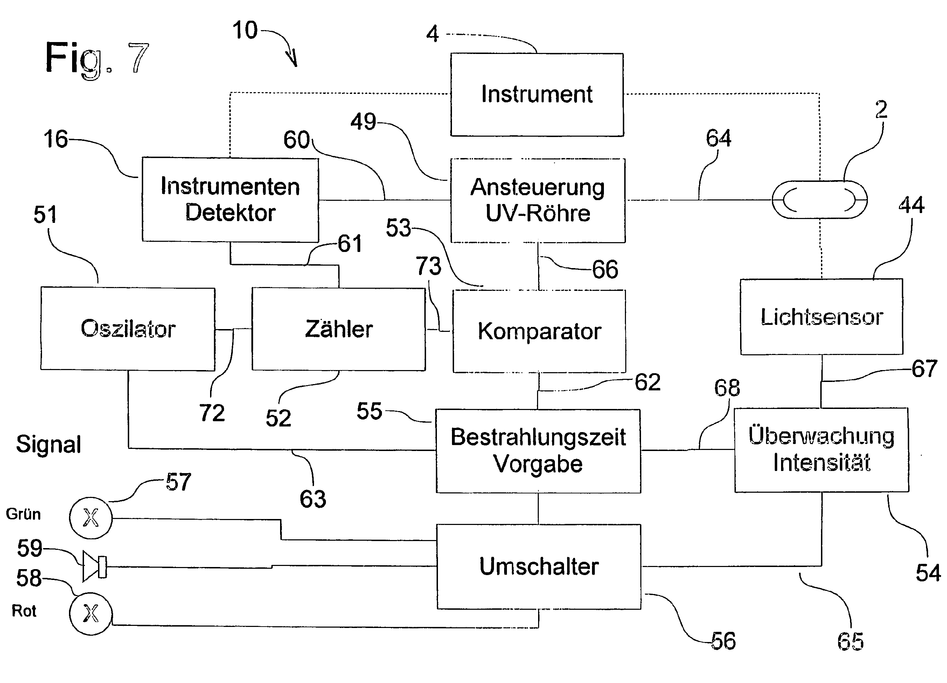

Das Blockschaltbild einer so ausgeführten Vorrichtung ist in Fig. 7 gezeigt. Dieses Blockschaltbild unterscheidet sich vom Blockschaltbild gemäss Fig. 4 eigentlich nur darin, dass die Einrichtung "Überwachung Intensität" 54 einen noch zweiten Ausgang hat, welcher an einen zweiten Eingang der Einrichtung "Bestrahlungszeitvorgabe" 55 angeschlossen ist. Über eine diesbezügliche Leitung 68 erhält die Einrichtung " Bestrahlungszeitvorgabe" 55 die Auskunft über die aktuelle Intensität der von der Röhre 2 abgegebenen UV-Lichtes.

Damit eine zur Entkeimung erforderliche, verlängerte Bestrahlungszeit bei abnehmender Intensität des durch die Röhre 2 abgegebenen Lichtes ermittelt werden kann, muss der Zusammenhang zwischen gemessener Strahlungsintensität und der verlängerten Bestrahlungszeit die Einrichtung " Bestrahlungszeitvorgabe" 55 eingegeben sein. Dieser Zusammenhang ist erfahrungsgemäss ein nichtlinearer Zusammenhang. Dieser Zusammenhang kann in einer Kennlinie modelliert. Diese Kennlinie ist in einem elektronischen Speicher derart gespeichert, dass zu jedem gemessen Wert der Strahlungsintensität eine dementsprechend verlängerte Bestrahlungszeit in der Einrichtung "Bestrahlungszeitvorgabe" 55 ermittelt werden kann.

Das dazu geeignete elektronische Speicherwerk kann entweder in Analogtechnik oder in Digitaltechnik ausgeführt sein. Bei einer Ausführung in Digitaltechnik ist das elektronische Speicherwerk ein PROM, EPROM oder eine funktionell vergleichbare Komponente. Dabei ist der digitalisierte Messwert der "Überwachung Intensität" die Eingangsgrösse an einem Adressbus der Komponente. Die Ausgangsgrösse ist die der verminderten Strahlungsintensität entsprechend verlängerte Bestrahlungszeit. Die neue Bestrahlungszeit wird über die Steuerleitung 62 dem entsprechenden Eingang des Komparators 53 zur Steuerung des Betriebes der Einrichtung "Ansteuerung UV-Röhre" 49 zugeführt.

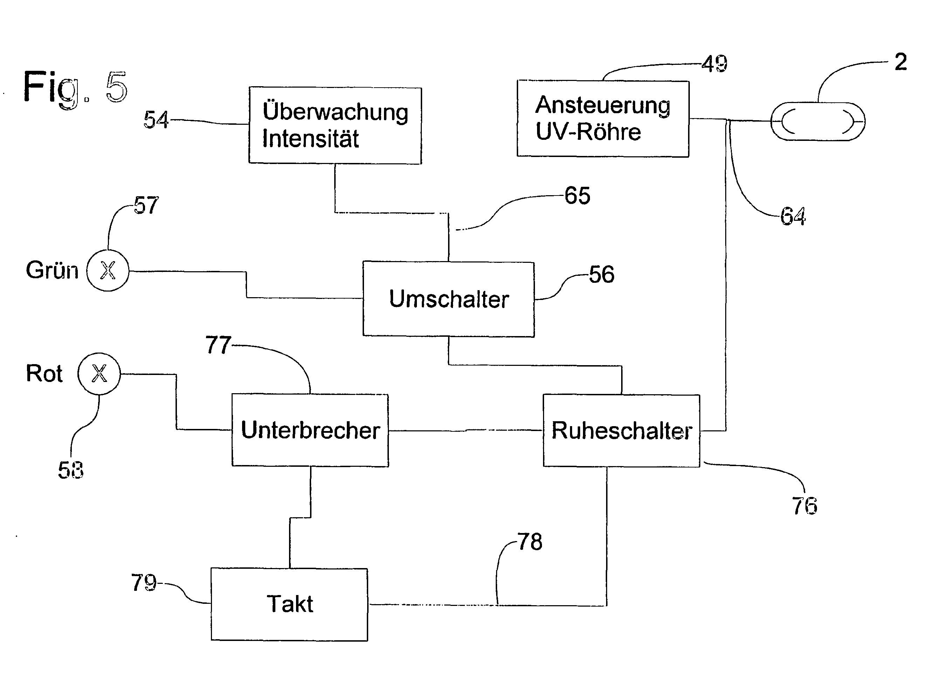

Fig. 5 zeigt eine Schaltungsanordnung, welche eine erste Möglichkeit, nämlich eine optische Anzeige der Betriebszustände der UV-Röhre 2 ermöglicht. An einen ersten Ausgang des Umschalters 56 ist eine erste Signallampe 57 angeschlossen. An einen zweiten Ausgang des Umschalters 56 ist eine zweite Signallampe 58 angeschlossen. Die erste Signallampe 57 kann im vorliegenden Fall grün leuchten und sie zeigt jenen Betriebszustand der UV-Röhre 2 an, während welchem die Instrumente 4 mit der vorgeschriebenen Intensität des UV-Lichtes entkeimt werden. Die zweite Signallampe 58 kann im vorliegenden Fall rot leuchten und sie zeigt jenen Betriebszustand der UV-Röhre 2 an, während welchem die Intensität des abgestrahlten UV-Lichtes unterhalb der vorgeschriebenen Intensität liegt.

Die genannten Lampen 57 und 58 können als eine einzige und an sich bekannte Diode 95 (Fig. 3) ausgeführt sein, welche in zwei verschiedenen Weisen angesteuert werden kann. Je nach der Art der Ansteuerung kann diese Diode 95 grün oder rot leuchten. Die Diode 95 ist auf der Oberseite des Trägers 20 montiert und sie ist empor gerichtet. In der Oberwand 80 des Frontdeckels 1 ist eine entsprechende Öffnung 96 so ausgeführt. Durch diese Öffnung 96 kann die Diode 95 beobachtet werden, mit welcher Farbe sie leuchtet, wenn sie zwischen grün und rot umschaltet. Solange die Intensität des UV-Lichtes unterhalb dem Schwellpegel im Block "Ueberwachung Intensität" 54 liegt, brennt die rote Lampe 58. Nachdem die Intensität des UV-Lichtes den Schwellpegel überschritten hat, brennt die grüne Lampe 57.

Damit die rote Signallampe 58 nicht weiter brennt, nachdem der Entkeimungsvorgang ordentlich beendet worden ist, bedarf es weiterer Schaltungselemente in der vorliegenden Vorrichtung (Fig. 5).

Wie bereits erwähnt, ist die grüne Lampe 57 an den ersten Ausgang des Umschalters 56 angeschlossen. An den zweiten Ausgang des Umschalters 56 ist einer der Eingänge eines Ruheschalters 76 angeschlossen. Ein zweiter Eingang des Ruheschalters 76 ist über eine Steuerleitung 65 an die Speiseleitung 64 und somit auch an den Ausgang der Einrichtung "Ansteuerung UV-Röhre" 49 angeschlossen. An den Ausgang des Ruheschalters 76 ist ein erster Eingang eines Unterbrechers 77 angeschlossen, an dessen Ausgang die rote Lampe 58 angeschlossen ist. An den Ausgang des Ruheschalters 76 ist zugleich ein Zeitglied 78 angeschlossen, dessen Ausgang an einen Steuereingang des Unterbrechers 77 angeschlossen ist.

Ist die Entkeimung erfolgreich und ordentlich beendet worden, so wird die Speisung der UV-Röhre 2 über die Speiseleitung 64 abgebrochen und diese erlischt. Dabei sinkt der Pegel des Signales in der Einrichtung "Überwachung Intensität" 54 unter den vorgesehen Schwellpegel, sodass der Umschalter 56 von der grünen Lampe 57 auf seinen zweiten Ausgang umschaltet, an welchen der erste Eingang des Ruheschalters 76 jetzt anstelle der rote Signallampe 58 angeschlossen ist. Ohne diesen Ruheschalter 76 würde die rote Signallampe 58 jetzt ununfierbrochen weiter leuchten. Das Ausbleiben des Signals auf der Speiseleitung 64 und somit auch am zweiten Eingang des Ruheschalters 76 wirkt sich so aus, dass der Ruheschalter 76 die Speisung der roten Signallampe 58 mit Energie unterbricht und die rote Lampe 58 ebenfalls erlischt oder gar nicht beginnt zu leuchten.

Der Ausfall der UV-Röhre 2 bei laufendem Betrieb, d.h. während einem Entkeimungsvorgang, wird von der Steuerelektronik 10 ebenfalls erkannt. Beim Ausfall der UV-Röhre 2 empfängt der Lichtsensor 44 kein Licht mehr von der UV-Röhre 2. Die Intensitätsüberwachung 54 erhält vom Lichtsensor 44 somit kein Signal, sodass der Pegel am Eingang der Intensitätsüberwachung 54 unterhalb der genannten Umschaltschwelle liegt. Die Intensitätsüberwachung 54 gibt ein entsprechendes Signal an den Umschalter 56 weiter, was den Umschalter 56 zum Umschalten vom Ausgang "grünes Licht" 57 zu seinem zweiten Ausgang, nämlich, zum ersten Eingang des Ruheschalters 76 veranlasst. Die Spannung auf der Speiseleitung 64 zur UV-Röhre 2 bleibt in diesem Fall jedoch erhalten, weil die Spannung an der Röhre 2 weiterhin angelegt ist. Dies nämlich ohne Rücksicht darauf, ob die Röhre 2 defekt ist oder nicht. Folglich liegt diese Spannung auch am zweiten Eingang des Ruheschalters 76.

Im Unterschied zum vorher genannten Fall, befindet sich jetzt die Speisespannung am zweiten Eingang des Ruheschalters 76. Für diesen Fall ist der Ruheschalter 76 so ausgeführt, dass er ein Signal zu einem Taktglied 79 sendet, welches den Unterbrecher 77 veranlasst, die Speisespannung zur roten Lampe 58 periodisch zu unterbrechen. Durch das blinkende rote Licht 58 wird dem Benützer der Vorrichtung signalisiert, dass die UV-Röhre defekt ist und dass sie zu ersetzen ist. Das rote Licht 58 blinkt, bis man den Stecker des Stecknetzgerätes 9 aus der Vorrichtung herauszieht und dadurch die Speisung der Vorrichtung beendet.

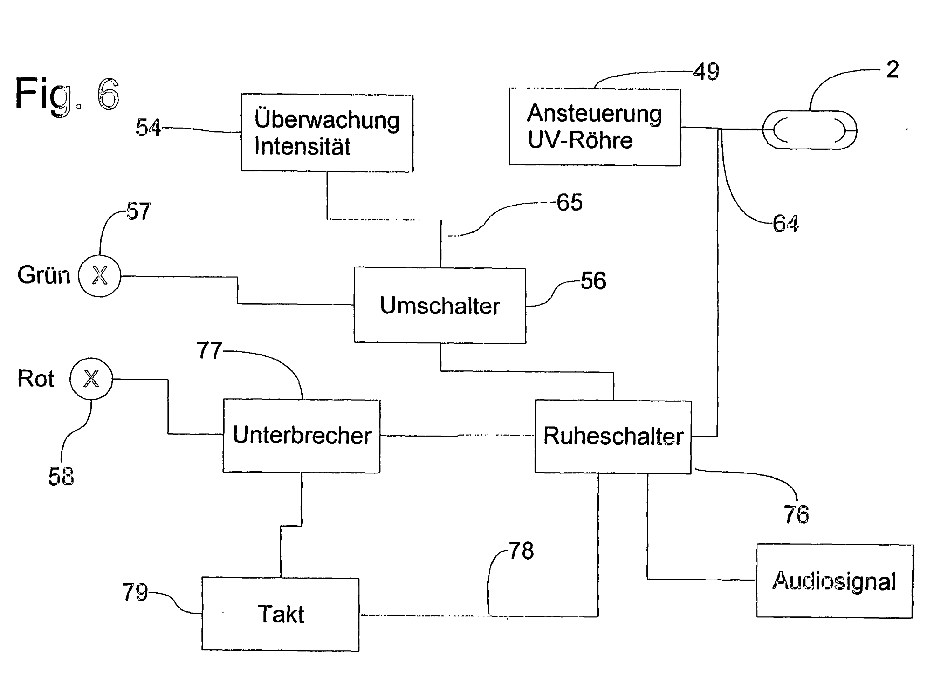

Fig. 6 zeigt ein Blockschaltbild einer zweiten Ausführungsmöglichkeit für die Signalisation der Betriebszustände der vorliegenden Vorrichtung. Dieses Blockschaltbild ähnelt weitest gehend dem Blockschaltbild aus Fig. 5. Im Unterschied zum Blockschaltbild gemäss Fig. 5 ist eine akustische Signaleinrichtung 59 an einen (weiteren) Ausgang des Ruheschalters 56 angeschlossen. Diese akustische Signaleinrichtung 59 kann einen Lautsprecher (nicht dargestellt) aufweisen. Die akustische Signalisierung kann dabei über einen Signaltongenerator oder einen Sprachsynthesizer mit vorgegebenen Sprachsequenzen erfolgen.

In einer weiteren Ausbildung der Steuerungseinrichtung kann dieselbe auch mit einem Thermostat (nicht dargestellt) ausgestattet sein, der die eingeschaltete UV-Lichtquelle beim Überschreiten einer vorgegebenen Temperatur, insbesondere im Inneren des Gehäuses 1. Der Anschluss des Thermostaten ist ausserdem so gestaltet, dass dieser die UV-Röhre wieder in Betrieb nimmt, nachdem die Temperatur unter die Schwelle der Gefährdung sang.

Die Vorrichtung zeichnet sich durch einen minimalen Stromverbrauch aus und arbeitet im für Badezimmer gefahrlosen Niederspannungsbereich. Diese beschriebene Vorrichtung zum Keimfreimachen und Keimfreihalten von Zahnbürsten ist nicht nur für private Haushalte gedacht, sie kann vielmehr auch in zahnärztlichen Praxen und in Hotelzimmern verwendet werden. Es liegt im Rahmen der Erfindung, die beschriebene Vorrichtung oder eine ähnlich aufgebaute Vorrichtung zum Keimfreimachen und Keimfreihalten von anderen Instrumenten, welche der Behandlung oder der Pflege des menschlichen oder tierischen Körpers dienen, zu verwenden. In Frage kommen dabei neben ärztlichen bzw. zahnärztlichen Instrumenten auch Coiffeur-Instrumente, wie Rasiermesser und dergleichen sowie Hand- und Fusspflege-Instrumente. Hierdurch schaltet sich die UV-Strahlungsquelle bei einem Bewegungsvorgang innerhalb des Gehäuses bzw. des Aufnahmebehälters automatisch ein, wodurch die Entkeimung eingeleitet wird.

Durch eine wissenschaftliche Untersuchung der erfindungsgemässen Vorrichtung durch das Institut für Hygiene und Medizinische Mikrobiologie der Universität Bern wurde deren Wirksamkeit festgestellt. Aus einer Übernachtkultur von Escherichia coli wurde eine Keimsuspension hergestellt, die rund 5 x 106 Keime pro ml enthielt. In diese Keimsuspension wurde dann eine Reihe von Zahnbürsten eingetaucht und anschliessend ausgeschlagen, um überschiessende Flüssigkeit mit Bakterien zu entfernen. Die so vorbehandelten Bürsten wurden dann entweder direkt in ein frisches und steriles Nährmedium gebracht, oder 10 Minuten, 20 Minuten oder 40 Minuten in die Vorrichtung bei brennender UV-Lichtquelle gestellt, wobei jeweils die Keimzahl in diesen Medien bestimmt wurde. Daraus ergab sich, dass nach 40 Minuten 98 % der Keime abgetötet waren. Auf Grundlage dieser Versuche ist deshalb die Steuerungseinrichtung vorteilhaft mit einem Zähler ausgestattet, welcher die eingeschaltete UV-Lichtquelle nach einer vorgegebenen Zeit, beispielsweise nach einer Stunde, selbsttätig abschaltet.

## Patentansprüche

1. Vorrichtung zum Entkeimen von Instrumenten (4), insbesondere von Zahnbürsten (6), durch ultraviolette Strahlung, welche durch eine Strahlungsquelle (2) erzeugt werden kann, wobei diese Vorrichtung so ausgeführt ist, dass der Betrieb der Strahlungsquelle (2) überwacht werden kann, mit einem Innenteil (7), welcher eine längliche Hinterwand (85) aufweist, über welche der Innenteil (7) an einer Wand angebracht sein kann, und mit einem Gehäuse (1), welches dem Innenteil zugeordnet sein kann, **dadurch gekennzeichnet, dass** der Innenteil (7) Wangen (25) aufweist, dass sich je eine dieser Wangen (25) an die jeweilige Endkante der länglichen Innenteil-Hinterwand (85) anschliesst und zur Ebene dieser Hinterwand (85) senkrecht steht, dass das Gehäuse (1) Seitenwände (81,82) aufweist und über diese Seitenwände an den Wangen des Innenteils (7) schwenkbar und einrastbar angebracht ist, dass ein länglicher Träger (20) für den elektronischen Teil (10) der vorliegenden Vorrichtung vorgesehen ist, welcher sich im Innenteil (7) befindet, dass die Endabschnitte (24) dieses länglichen Trägers (20) je eine Buchse (42,43) für die Aufnahme des Stekkers eines Netzteils (9) tragen, dass die Gehäuseseitenwände (81,82) mit je einer Öffnung (40,41) versehen sind und dass diese Öffnungen so ausgeführt sind, dass der Stecker einer Niederspannungsquelle (9) durch die betreffende Öffnung (40,41) in der Gehäuseseitenwand hindurchgeführt und in die betreffende Buchse (42,43) gesteckt sein kann, wenn sich das Gehäuse (1) im geschlossenen Zustand befindet.

2. Vorrichtung nach Patentanspruch 1, **dadurch gekennzeichnet, dass** die Längsachsen der Buchsen (42,43) für die Aufnahme des Steckers der Niederspannungsque!le (9) parallel zur Ebene des Grundkörpers (21) des Trägers (20) verlaufen, dass sich die Buchsen (42,43) in einander gegenüberliegenden Richtungen öffnen, und zwar nach aussen, und dass je eine der Öffnungen (40, 41) in der Gehäuseseitenwand (81,82) mit der Aufnahmemündung einer der Buchsen (42,43) fluchtet.

3. Vorrichtung nach Patentanspruch 2, **dadurch gekennzeichnet, dass** eine Trennwand (33) vorgesehen ist, welche sich in einem Abstand von der Hinterwand (85) des Innenteils (7) befindet, dass der längliche Träger (20) für den elektrischen Teil (10) in einem Aufnahmeschacht (35) angeordnet ist, welcher sich zwischen der Hinterwand (85) des Innenteils (7) und der Trennwand (33) befindet, dass der Träger (20) eine längliche Strahlungsquelle (2) trägt, dass eine längliche Öffnung (34) in der Trennwand (35) ausgeführt ist, welche sich parallel zur Strahlungsquelle (2) erstreckt, und dass eine Halteeinrichtung (90) für die zu behandelnden Instrumente (4) vorgesehen, welche der gegenüber liegenden Seite der Trennwand (33) zugeordnet ist.

4. Vorrichtung nach Patentanspruch 3, **dadurch gekennzeichnet, dass** der Grundkörper (91) der Halteeinrichtung (90) einen im wesentlichen L-förmigen Querschnitt hat, dass die Einrichtung (90) ferner eine längliche Wanne (94) sowie einen Kamm (17) umfasst, dass sich die Wanne (94) auf dem horizontalen L-Schenkel (92) befindet, dass sich eine schlitzartige Führung (29) vom Boden der Wanne (94) aufwärts erstreckt, dass die einander gegenüber liegenden Randpartien (32) des Kammes (17) in die jeweilige Führung (29) eingesetzt sind, und dass der Kamm (17) Einschnitte (19) aufweist, welche von oben her vertikal verlaufen, und dass der jeweilige Einschnitt (1) sich gegen eine von Öffnungen (3) öffnet, welche in der Oberwand (80) des Behälters (1) ausgeführt sind.

5. Vorrichtung nach Patentanspruch 3, **dadurch gekennzeichnet, dass** ein Sensor (44) der Strahlungsquelle (2) zugeordnet ist, welcher den Betrieb dieser Strahlungsquelle (2) überwachen kann, wobei dieser Sensor ein Tageslichtsensor sein kann, dass an den Ausgang des Sensors (44) eine Einrichtung (54) zur Messung der Intensität der von der Strahlungsquelle (2) abgegebenen Strahlung angeschlossen ist, dass Anzeigelampen (57,58) und/oder ein akustischer Signalgeber (59) an getrennte Ausgänge der Einrichtung (54) zur Messung der Intensität der von der Strahlungsquelle (2) abgegebenen Strahlung angeschlossen sind und dass der elektrische Teil (10) einen Abschnitt umfasst, welcher zur Steuerung der Brenndauer der Strahlungsquelle (2) dient.

6. Vorrichtung nach Anspruch 5, **dadurch gekennzeichnet, dass** eine Öffnung (96) in der Oberwand (80) des Gehäuses (1) ausgeführt ist und dass sich zumindest eine der Anzeigelampen (57,58) unterhalb dieser Öffnung (80) befindet, sodass der Zustand dieser durch die Öffnung (80) beobachtet werden kann.

7. Vorrichtung nach Anspruch 5, **dadurch gekennzeichnet, dass** der elektrische Teil (10) einen Oszillator (51) aufweist, welcher über einen Zähler (52) an einen der Eingänge eines Komparators (53) angeschlossen ist, dass eine Einrichtung (55) zur Bestimmung der Bestrahlungszeit durch die Strahtungsquelle (2) vorgesehen ist, dass der Ausgang dieser Bestimmungseinrichtung (55) an einen zweiten Eingang des Komparators (53) angeschlossen ist, dass der Ausgang des Komparators (53) an einen zweiten Eingang der Ansteuerungseinrichtung (49) angeschlossen ist und dass ein zweiter Ausgang des Instrumentendetektors (16) an einen zweiten Steuereingang des Zählers (52) angeschlossen ist.

8. Vorrichtung nach Anspruch7, **dadurch gekennzeichnet, dass** ein zweiter Ausgang (68) der Einrichtung (54) zur Überwachung der Intensität an einen Steuereingang der Einrichtung (55) zur Bestimmung der Bestrahlungszeit angeschlossen ist.

9. Vorrichtung nach Patentanspruch 7, **dadurch gekennzeichnet, dass** ein Detektor (16) für die Einführung der Instrumente (4) in die Vorrichtung vorgesehen ist, dass dieser Detektor (16) der Bewegungsbahn der Instrumente (4) zugeordnet ist, welche in das Gehäuse (1) eingeführt werden, und dass der Ausgang dieses Detektors (16) an eine Einrichtung zur Ansteuerung (49) der Strahlungsquelle (2) angeschlossen ist.

10. Vorrichtung nach Patentanspruch 5, **dadurch gekennzeichnet, dass** der Sensor (16) als eine Lichtschranke ausgeführt ist, dass diese Lichtschranke einen Lichtsender (161) und einen Lichtempfänger (162) umfasst, zwischen welchen sich eine Lichtstrecke (163) befindet, dass die Lichtquelle (161) und der Lichtempfänger (162) an je einem Halter (23) befestigt sind, welche sich in einem Abstand voneinander und oberhalb der Platine (20) befinden, und dass die Lichtschranke (16) oberhalb jenem Raum angeordnet ist, in welchem sich die Köpfe der in die Vorrichtung eingeführten Instrumente (4) während der Entkeimung befinden.

11. Vorrichtung nach Patentanspruch 1, **dadurch gekennzeichnet, dass** zwei Fassungen (22) für die Endpartien der länglichen Strahlungsquelle (2) auf der Unterseite des Grundkörpers (21) des Träger (20) befestigt sind, dass diese Fassungen (22) so angeordnet sind, dass sich die Strahlungsquelle (2) auf der Höhe des Kopfes (5) der Instrumente (4) befindet, welche in der Vorrichtung eingesteckt sind.

## Claims

1. Device for the sterilizing of instruments (4), in particular of toothbrushes (6), by aid of ultra-violet radiation which can be produced by a radiation source (2), whereby this device is constructed in such a way, that the operation of the radiation source (2) can be monitored, having an inside part (7) which is provided with an elongated rear wall (85) over which this inside part (7) can be attached to a wall, and having a case (1), which is assigned to the inside part, **characterized** thereby that this inside part (7) shows cheeks (25), that each one of these cheeks (25) is assigned to the respective trailing edge of the elongated inside part (7) of the rear wall (85), whereby these cheeks (25) stand perpendicularly to the plane of this rear wall (85), that the case (1) has side walls (81,82), that the case (1) is assigned by aid of said side walls to the cheeks of the inside part (7) in a swingable and locking manner, that an elongated carrier (20) is provided for which is placed in the inside part (7), that the end sections (24) of this elongated carrier (20) is provided with a socket (42,43) for a plug of a power supply (9), that the side walls (81,82) of the case are provided with openings (40,41) and that these openings are carried out in such a way that the plug of a low voltage source (9) can pass through the respective opening (40,41) in the side wall of the case and that it can be plugged into the respective socket (42,43) when the case (1) is closed

2. Device according to patent claim 1, **characterized** thereby that the longitudinal axes of the sockets (42,43) for the insertion of the plug of the low voltage source (9) extend parallel to the plane of the main body (21) of the carrier (20), that the sockets (42,43) are opened in opposite directions to each other, i.e. to the outside, and that each one of the openings (40, 41) in the side walls (81, 82) of the housing is in alignment with the mouth of one of the sockets (42, 43).

3. Device according to patent claim 2, **characterized** thereby that a partition (33) is provided for, which is in a distance of the rear wall (85) of the inside part (7), that the elongated carrier (20) for the electrical part (10) is located in a receiving compartment (35) which is placed between the rear wall (85) of the inside part (7) and the partition (33), that the carrier (20) bears an elongated radiation source (2), that an elongated opening (34) is carried out in the partition (35) which extends parallel to the radiation source (2), and that a holding equipment (90) for the treated instruments (4) is provided for which is assigned to the opposite lying side of the partition (33).

4. Device according to patent claim 3, **characterized** thereby that the basic body (91) of the holding equipment (90) has a basically L-shaped cross-section, that the equipment (90) further comprises an elongated tub (94) as well as a comb (17), that the tub (94) is placed on the horizontal arm (92), that a slot-like guide means (29) extends from the ground of the tub (94) upwards, that the edge parts (32) of the comb (17) which lay opposite to each other are set in the respective guide means (29), and that the comb (17) shows incisions (19), which run vertically downstairs, and that the respective incision (19) opens against one of openings (3), which are carried out in the upper wall (80) of the receptacle (1).

5. Device according to patent claim 3, **characterized** thereby that a sensor (44) is assigned to the radiation source (2), which can monitor the operation of this radiation source (2) whereby said sensor can be a daylight sensor, that an equipment (54) for the measurement of the intensity of the radiation emitted from the radiation source (2) is attached to the exit of the sensor (44), that indicator lamps (57,58) and/or an acoustic signaler (59) are connected to separate exits of the equipment (54) for the measurement of the intensity of the radiation emitted from the radiation source (2) and that the electrical part (10) comprises a section, which serves for controlling of the operation time of the radiation source (2).

6. Device according to patent claim 5, **characterized** thereby that an opening (96) is carried out in the upper wall (80) of the case (1) and that at least one of the indicator lamps (57,58) is placed below of this opening (80) so that the state of this indicator lamp (57,58) can be observed through the opening (80).

7. Device according to patent claim 5, **characterized** thereby that the electrical part (10) comprises an oscillator (51) which is connected through a counter (52) onto one of the inputs of a comparator (53), that an equipment (55) for the determining of the time for the exposure to the radiation from the radiation source (2), that the output of this equipment (55) is connected to a second input of the comparator (53), that the output of the comparator (53) is connected to a second input of the control equipment (49), and that a second input of the instrument detector (16) is connected to a second control input of the counter (52).

8. Device according to patent claim 7, **characterized** thereby that a second exit (68) of the equipment (54) for the monitoring of the intensity is connected to a control input of the equipment (55) for the determining of the radiation time.

9. Device according to patent claim 7, **characterized** thereby that a detector (16) establishing the insertion of the instruments (4) into the device is provided for, that this detector (16) is assigned to the movement path of the instruments (4) which are inserted into the case (1), and that the output of this detector (16) is connected to an equipment (49) for the controlling of the radiation source (2).

10. Device according to patent claim 5, **characterized** thereby that the sensor (16) is carried out as a light barrier, that this light barrier contains a phototransmitter (161) and a light detector (162) whereby a light path (163) is placed therebetween, that the source of light (161) and the light detector (162) are attached to a respective holder (23) which are placed in a distance of each other and above the board (20), and that the light barrier (16) is placed above that space in which the heads of the instruments (4) introduced into the device are located during the sterilization thereof.

11. Device according to patent claim 1, **characterized** thereby that two holder (22) for the end portions of the elongated radiation source (2) are secured to the underside of the basic body (21) of the carrier (20), that these holders (22) are placed in such a way that the radiation source (22) is arranged on the heightof the heads (5) of the instruments (4) which are inserted in the device.

## Revendications

1. Appareil destiné à la stérilisation d'instruments (4), en particulier de brosses à dents (6), par rayonnement ultraviolet, qui peut être produit par une source de rayonnement (2), cet appareil étant conçu de telle sorte que le fonctionnement de la source de rayonnement (2) peut être surveillé, avec une partie intérieure (7) qui présente une paroi arrière longitudinale (85) sur laquelle la partie intérieure (7) peut être montée sur une paroi, et avec un boîtier (1) qui peut être affecté à la partie intérieure, **caractérisé en ce que** la partie intérieure (7) présente des faces (25), **en ce que** chacune de ces faces (25) se raccorde au bord extrême respectif de la paroi arrière longitudinale (85) de la partie intérieure et se trouve perpendiculaire au plan de cette paroi arrière (85), **en ce que** le boîtier (1) présente des parois latérales (81, 82) et est disposé sur ces parois latérales de façon orientable et verrouillable sur les faces de la paroi intérieure (7), **en ce qu'**un support longitudinal (20) est prévu pour la partie électronique (10) du présent appareil qui se trouve dans la partie intérieure (7), **en ce que** les sections finales (24) de ce support longitudinal (20) portent respectivement une prise (42, 43) pour la réception du connecteur d'un bloc de secteur (9), **en ce que** les parois latérales du boîtier (81, 82) sont munies respectivement d'une ouverture (40, 41) et **en ce que** ces ouvertures sont conçues de telle sorte que le connecteur d'une source à basse tension (9) passe à travers l'ouverture (40, 41) correspondante dans la paroi latérale du boîtier et peut être connecté dans la prise (42, 43) correspondante, lorsque le boîtier (1) se trouve à l'état fermé.

2. Appareil selon la revendication 1, **caractérisé en ce que** les axes longitudinaux des prises (42, 43) pour la réception du connecteur de la source à basse tension (9) sont parallèles au plan du corps de base (21) du support (20), **en ce que** les prises (42, 43) s'ouvrent dans des directions opposées l'une à l'autre et plus précisément vers l'extérieur et **en ce que** chacune des ouvertures (40, 41) dans la paroi latérale du boîtier (81, 82) est alignée avec l'embouchure de réception d'une des prises (42, 43).

3. Appareil selon la revendication 2, **caractérisé en ce qu'**une paroi de séparation (33) est prévue et se trouve à une certaine distance de la paroi arrière (85) de la partie intérieure (7), **en ce que** le support longitudinal (20) pour la partie électrique (10) est disposé dans une gaine de réception (35) qui se trouve entre la paroi arrière (85) de la partie intérieure (7) et la paroi de séparation (33), **en ce que** le support (20) porte une source de rayonnement longitudinale (2), **en ce qu'**une ouverture longitudinale (34) est réalisée dans la paroi de séparation (35) qui s'étend parallèlement à la source de rayonnement (2) et **en ce qu'**un dispositif de fixation (90) est prévu pour les instruments à traiter (4) et est attribué au côté opposé de la paroi de séparation (33).

4. Appareil selon la revendication 3, **caractérisé en ce que** le corps de base (91) du dispositif de fixation (90) possède une section pour l'essentiel en forme de L, **en ce que** le dispositif (90) comprend en outre une coque longitudinale (94) ainsi qu'un peigne (17), **en ce que** la coque (94) se trouve sur la branche horizontale du L, **en ce qu'**un guidage en forme de fente (29) s'étend vers le haut depuis le fond de la coque (94), **en ce que** les parties de bordure (32) du peigne (17) opposées sont insérées dans le guidage respectif (29) et **en ce que** le peigne (17) présente des incisions (19) qui s'étendent verticalement depuis le haut et **en ce que** chaque incision respective (19) s'ouvre vers une des ouvertures (3) qui sont réalisées dans la paroi supérieure (80) du conteneur (1).

5. Appareil selon la revendication 3, **caractérisé en ce qu'**un capteur (44) est associé à la source de rayonnement (2) et peut surveiller le fonctionnement de cette source de rayonnement (2), ledit capteur pouvant être un capteur de la lumière du jour, **en ce qu'**un dispositif (54) destiné à la mesure de l'intensité du rayonnement émis par la source de rayonnement (2) est raccordé à la sortie du capteur (44), **en ce que** des voyants (57, 58) et/ou un émetteur de signal acoustique (59) sont connectés aux sorties séparées du dispositif (54) pour mesurer l'intensité du rayonnement émis par la source de rayonnement (2) et **en ce que** la partie électrique (10) comprend une section qui sert à piloter la durée d'émission de la source de rayonnement (2).

6. Appareil selon la revendication 5, **caractérisé en ce qu'**une ouverture (96) est réalisée dans la paroi supérieure (80) du boîtier (1) et **en ce qu'**au moins un des voyants (57, 58) se trouve sous cette ouverture (80) de sorte que l'état de celui-ci puisse être observé par l'ouverture (80).

7. Appareil selon la revendication 5, **caractérisé en ce que** la partie électrique (10) présente un oscillateur (51) qui est connecté par un compteur (52) à une des entrées d'un comparateur (53), **en ce qu'**un dispositif (55) destiné à la détermination du temps de rayonnement de la source de rayonnement (2) est prévu, **en ce que** la sortie de ce dispositif de détermination (55) est connectée à une deuxième entrée du comparateur (53), **en ce que** la sortie du comparateur (53) est connectée à une deuxième entrée du dispositif de commande (49) et **en ce qu'**une deuxième sortie du détecteur d'instruments (16) est connectée à une deuxième entrée de commande du compteur (52).

8. Appareil selon la revendication 7, **caractérisé en ce qu'**une deuxième sortie (68) du dispositif (54) destiné à contrôler l'intensité est connectée à une entrée de commande du dispositif (55) de détermination du temps de rayonnement.

9. Appareil selon la revendication 7, **caractérisé en ce qu'**un détecteur (16) est prévu pour l'introduction des instruments (4) dans l'appareil, **en ce que** ce détecteur (16) est attribué au parcours des instruments (4) qui sont introduits dans le boîtier (1) et **en ce que** la sortie de ce détecteur (16) est connectée à un dispositif destiné à la commande (49) de la source de rayonnement (2).

10. Appareil selon la revendication 5, **caractérisé en ce que** le capteur (16) est conçu comme une barrière lumineuse, **en ce que** cette barrière lumineuse comprend un émetteur de lumière (161) et un récepteur de lumière (162) entre lesquels se trouve une trajectoire de lumière (163), **en ce que** la source de lumière (161) et le récepteur de lumière (162) sont fixés sur des supports respectifs (23) qui se trouvent à une certaine distance l'un de l'autre et au-dessus de la platine (20) et **en ce que** la barrière lumineuse (16) est disposée au-dessus de chaque espace dans lequel se trouvent les têtes des instruments (4) introduits dans l'appareil pendant la stérilisation.

11. Appareil selon la revendication 1, **caractérisé en ce que** deux montures (22) pour les parties extrêmes de la source de rayonnement longitudinale (2) sont fixées sur la face inférieure du corps de base (21) du support (20), **en ce que** ces montures (22) sont disposées de telle sorte que la source de rayonnement (2) se trouve à la hauteur de la tête (5) des instruments (4) qui sont introduits dans l'appareil.
